# FASCICULE DE BREVET EUROPEEN

(11) **EP 1 185 210 B1**
(45) Date de publication et mention de la délivrance du brevet: **13.04.2005**
(21) Numéro de dépôt: 00942172.8
(22) Date de dépôt: 09.06.2000
(51) Int. Cl.: A61B 17/80

(54) **DISPOSITIF ANTI-RECUL POUR IMPLANT ORTHOPEDIQUE**
RÜCKLAUFSPERRE FÜR ORTHOPÄDISCHES IMPLANTAT
ANTI-SLIP DEVICE FOR AN ORTHOPEDIC IMPLANT

(30) Priorité: 17.06.1999 FR 9907884
(43) Date de publication de la demande: 13.03.2002
(73) Titulaire: EUROSURGICAL, 62217 Beaurains (FR)
(72) Inventeur: MAZEL, Christian, F-92220 Vaucresson (FR); VIART, Guy, F-62128 Saint Léger (FR)
(74) Mandataire: Schmitt, John
(86) Numéro de dépôt international: PCT/FR2000/001596
(87) Numéro de publication internationale: WO 2000/078238

(56) Documents cités:
- EP-A- 0 599 640
- DE-A- 4 409 833
- US-A- 5 876 402
- US-A- 5 879 389

## Description

La présente invention est relative à un dispositif anti-recul destiné à maintenir une vis de fixation dans un alésage préalablement percé dans l'épaisseur de la paroi d'un implant orthopédique.

On connaît des dispositifs de ce genre qui sont généralement adaptés à la forme de l'implant orthopédique, comme par exemple, des capots de protection qui viennent se fixer sur l'implant pour empêcher le recul des vis d'une part, et protéger l'environnement extérieur d'autre part.

On constate que ce genre de dispositif ne peut pas s'adapter à tous les types d' implants orthopédiques.

On connaît également des vis de fixation pour implant orthopédique qui comportent une tête de serrage à bord périphérique segmenté et au milieu de laquelle est percé un logement fileté prévu pour recevoir un bouchon. Ce dernier permet d'écarter le bord périphérique segmenté de la tête de serrage pour qu'elle vienne en appui contre la paroi interne de l'alésage de l'implant pour bloquer la vis de fixation.

On remarque que ce type de vis ne peut être utilisé que pour certains implants qui comportent des alésages de diamètre interne suffisant pour recevoir la tête de la vis. En effet, cette dernière présente un diamètre externe de sa tête qui est supérieur à celui des vis généralement utilisées pour la fixation des implants. De plus, ce dispositif de blocage réalisé par l'intermédiaire d'un bouchon rapporté dans la tête de la vis, empêche toute inclinaison angulaire de ladite vis et tout déplacement de cette dernière par rapport au corps de l'implant.

On connaît d'après le brevet US 5876402 un ensemble polyaxial de verrouillage comprenant une vis et une plaque destinées à immobiliser des os par fixation dans ceux-ci et notamment dans les corps vertébraux d'une colonne vertébrale.

La plaque présente des trous traversants à profil conique dans lesquels sont introduits respectivement une vis de fixation à tête sphérique et un élément conique de couplage venant autour de la tête de la vis et en appui sur les bords conique du trou. Chaque trou traversant présente dans sa partie supérieure et au dessus de l'élément conique de couplage une rainure coaxiale destinée à recevoir un élément de verrouillage formé d'une bride annulaire fendue. La bride fendue est centrée autour de l'axe du trou à profil conique empêchant le recul de l'élément de couplage conique.

On connaît d'après le brevet US 5879389 une prothèse de genou dont le plateau tibial est fixé dans l'os par l'intermédiaire de vis d'ancrage logées dans des trous traversant ledit plateau. Il est prévu dans l'alésage supérieur du trou recevant la tête de chaque vis une rainure coaxiale destinée à recevoir une bride annulaire fendue permettant d'empêcher le recul de la vis.

L'Implant orthopédique suivant la présente invention comprend au moins un alésage destiné à recevoir une vis de fixation solidaire d'une tête et un dispositif anti-recul comprenant :
- au moins un logement qui est décalé axialement par rapport aux axes principaux XX' et YY' de l'alésage,
- et au moins un moyen de retenue qui coopère avec le logement correspondant afin que ledit moyen de retenue traverse partiellement l'alésage correspondant recevant la vis de fixation pour pouvoir se déformer élastiquement sous un effort de pression afin de permettre le passage et la mise en place de la vis dans son alésage, tandis que le moyen de retenue revient dans une position d'origine, non déformée, lors du retrait de l'effort de pression, pour se positionner au-dessus de la tête de la vis afin d'empêcher cette dernière de se déplacer par rapport à l'implant suivant une direction parallèle à l'axe longitudinal de l'alésage.

L'Implant orthopédique suivant la présente invention comprend un dispositif anti-recul pourvu, dans un même plan horizontal, d'un logement de faible diamètre comportant un espace de débattement débouchant à l'intérieur de l'alésage et d'un moyen de retenue qui est constitué d'une tige dont une partie est fixée dans le logement de manière que l'autre partie traverse partiellement l'alésage et vienne déboucher à l'intérieur de l'espace de débattement pour se déformer élastiquement en flexion sous un effort de pression.

L'Implant orthopédique suivant la présente invention comprend une tige qui traverse l'alésage de l'implant suivant une direction qui est éloignée du point d'intersection des axes XX' et YY'.

L'Implant orthopédique suivant la présente invention comprend une tige, un trou et un espace qui sont disposés dans un même plan horizontal perpendiculaire à l'axe longitudinal de l'alésage.

L'Implant orthopédique suivant la présente invention comprend dans un même plan horizontal, un logement qui est formé d'une rainure circulaire décalée axialement par rapport aux axes principaux XX' et YY' de l'alésage de manière à ce que le moyen de retenue constitué d'une rondelle ouverte traverse partiellement l'alésage.

L'Implant orthopédique suivant la présente invention comprend une position du logement circulaire qui est prévue pour déboucher dans au moins deux alésages afin de permettre aux moyens de retenue constitués d'une rondelle ouverte de traverser partiellement les deux alésages.

L'Implant orthopédique suivant la présente invention comprend une rondelle ouverte qui est disposée dans le logement de manière à ce que son ouverture soit placée au-dessus de l'un des alésages pour pouvoir se déformer élastiquement.

L'Implant orthopédique suivant la présente invention comprend dans un même plan horizontal, un logement qui est formé d'un espace dont les bords opposés sont inclinés pour présenter un profil en forme de V et un moyen de retenue qui est constitué d'une tige de même profil introduite dans l'espace en forme de V pour se déformer élastiquement en flexion sous un effort de pression.

L'Implant orthopédique suivant la présente invention comprend une tige qui est disposée à l'intérieur de l'espace en forme de V de manière à ce que, de part et d'autre du point d'insertion des axes XX' et YY', l'alésage soit traversé partiellement par les branches inclinées de ladite tige.

L'Imptant orthopédique suivant la présente invention comprend dans un même plan horizontal, un logement qui est formé de deux trous parallèles débouchant à l'intérieur de l'un au moins des alésages de l'implant et un moyen de retenue qui est constitué de deux tiges coopérant respectivement avec les trous parallèles pour se déformer élastiquement en flexion sous un effort de pression.

L'Implant orthopédique suivant la présente invention comprend des trous parallèles l'un à l'autre et qui sont prévus pour traverser partiellement l'un au moins des alésages de chaque côté du point d'intersection des axes principaux XX' et YY' et suivant une direction parallèle à celle de l'axe YY'.

L'Imptant orthopédique suivant la présente invention comprend des trous parallèles l'un à l'autre et qui sont prévus pour traverser partiellement l'un au moins des alésages de chaque côté du point d'intersection des axes principaux XX' et YY' et suivant une direction parallèle à celle de l'axe XX'.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figures 1 et 2 sont des vues de dessus illustrant un alésage d'un implant orthopédique comportant un dispositif anti-recul constitué d'une tige élastique unilatérale.
Figure 3 est une vue représentant une première variante du dispositif anti-recul qui est constitué d'une rondelle élastique ouverte.
Figure 4 est une vue semblable à celle de figure 1, mais montrant une seconde variante du dispositif anti-recul qui est constitué d'une tige élastique en forme de V.
Figures 5 et 6 sont des vues représentant une troisième et quatrième variante du dispositif anti-recul qui est constitué de tiges élastiques bilatérales.

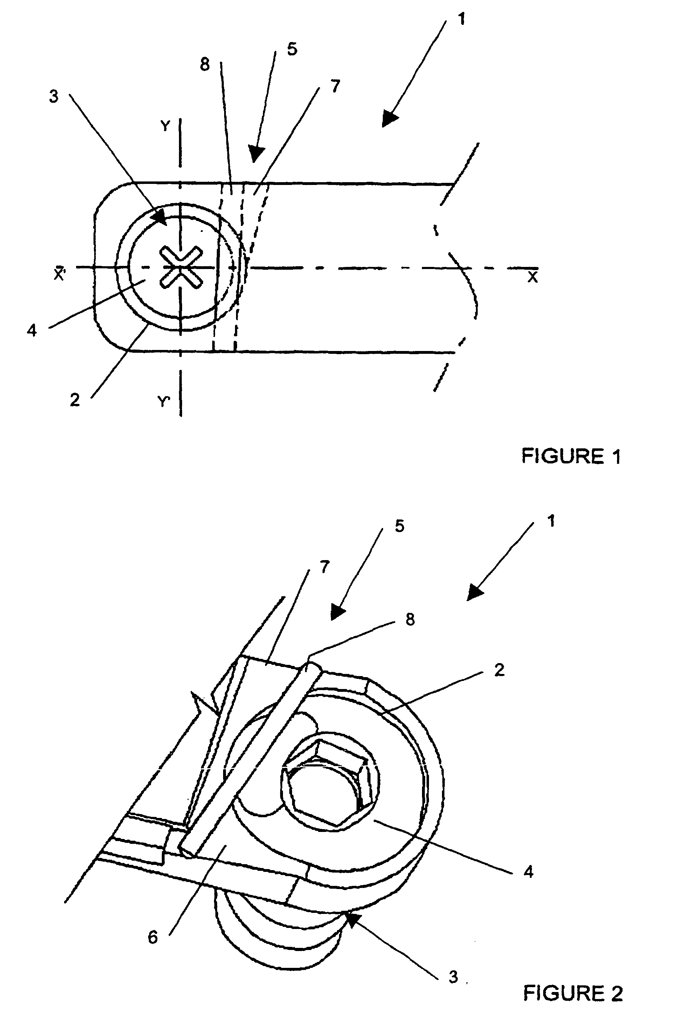

On a montré en figures 1 et 2 une partie d'un implant orthopédique 1, et plus particulièrement une portion de sa paroi dans laquelle est percé un alésage 2 prévu pour recevoir une vis 3 permettant la fixation de l'implant contre l'os d'un patient.

La vis de fixation 3 est prévue pour traverser l'alésage 2 de manière que sa tête de serrage 4 vienne se loger dans une partie de l'alésage destiné à cet effet. La vis de fixation 3 peut être positionnée à l'intérieur de l'alésage 2 suivant des directions angulaires précises par rapport à l'axe longitudinal dudit alésage pour venir s'ancrer dans la partie de l'os la plus résistante.

L'implant orthopédique 1 comporte au-dessus de la tête de serrage 4 de la vis de fixation 3 un dispositif anti-recul 5 empêchant à ladite vis, ancrée dans l'os, de se déplacer en translation par rapport à l'implant, et plus particulièrement suivant une direction parallèle à l'axe longitudinal de l'alésage 2.

Le dispositif anti-recul 5 comprend un logement qui est constitué, dans un même plan horizontal, d'un trou ou logement 6 de faible diamètre débouchant à l'intérieur de l'alésage 2 et d'un espace de débattement 7 situé dans le prolongement du trou 6.

Le trou ou logement 6 et l'espace 7 sont prévus sur le côté de l'alésage 2, c'est à dire qu'ils sont décalés par rapport au centre d'intersection des axes principaux XX' et YY' dudit alésage.

En effet, dans notre exempte de réalisation, l'alésage 2 est porté par les deux axes principaux XX' et YY' et un troisième axe dit « axe longitudinal » qui est perpendiculaire aux deux autres, ou perpendiculaire à un plan horizontal délimité par la surface circulaire de l'alésage.

Le dispositif anti-recul 5 comprend à l'intérieur du logement 6, 7 un moyen de retenue qui est constitué d'une tige 8 susceptible de se déformer élastiquement en flexion sous un effort de pression.

La tige 8 comporte une partie qui est fixée dans le trou ou logement 6 de manière que l'autre partie traverse l'alésage 2 et vienne déboucher à l'intérieur de l'espace de débattement 7.

La tige 8, le trou 6 et l'espace 7 du dispositif anti-recul 5 sont disposés dans un même plan horizontal qui est perpendiculaire à l'axe longitudinal de l'alésage 2.

La tige 8 traverse l'alésage 2 à l'intérieur de sa surface circulaire, mais en bordure de cette dernière.

Lors de l'introduction de la vis de fixation 3 à l'intérieur de l'alésage 2, et de son ancrage dans l'os suivant une direction angulaire déterminée, l'effort de pression soumis à la tige 8 par la tête de serrage 4 déforme en flexion ladite tige à l'intérieur de l'espace de débattement 7 pour laisser le passage à ladite vis.

On note, lorsque la vis 3 est complètement introduite dans l'alésage 2 ou ancrée dans l'os du patient, que la tige 8, du fait de son élasticité et du retrait de l'effort de pression, reprend sa position d'origine, non déformée, au-dessus de la tête de serrage 4 pour empêcher ladite vis de se déplacer par rapport à l'implant 1 suivant une direction sensiblement parallèle à celle de l'axe longitudinal de l'alésage.

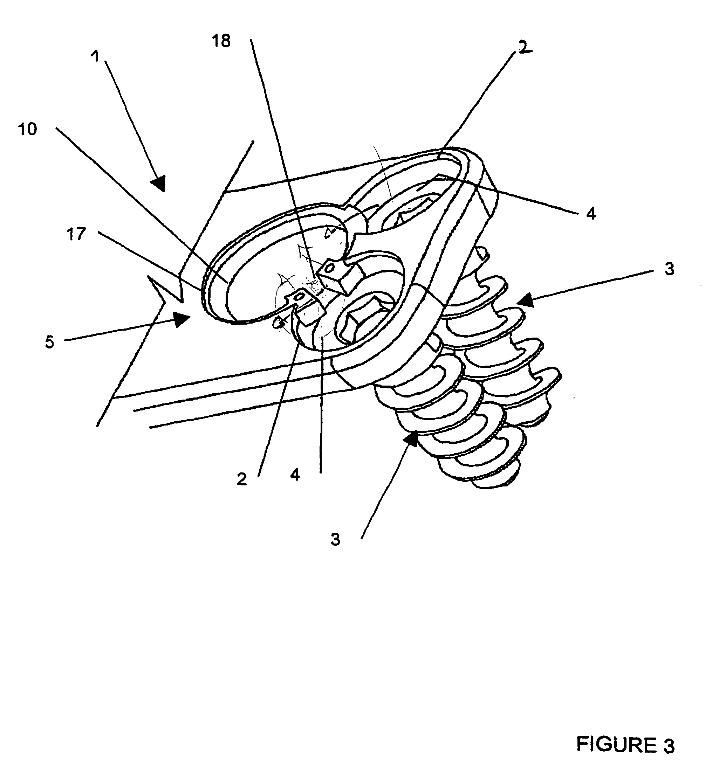

On a montré en figure 3 une première variante du dispositif anti-recul 5 suivant la présente invention.

L'implant orthopédique 1 est percé de deux alésages 2 prévus pour recevoir chacun une vis de fixation.

La partie de l'alésage 2 recevant la tête 4 de chaque vis de fixation 3 débouche dans un logement circulaire 17 qui est décalé axialement par rapport aux alésages 2 et qui est susceptible de recevoir une rondelle ouverte 10 afin de constituer le dispositif anti-recul 5. On constate que la rondelle ouverte 10 traverse partiellement chaque alésage 2 pour venir au-dessus de la tête 4 de chaque vis 3 après introduction.

La rondelle ouverte 10 est susceptible de se déplacer dans le logement circulaire 17 de manière que l'ouverture 18 puisse se présenter, soit au dessus du premier soit du second alésage.

Lors de l'introduction d'une vis de fixation 3 dans un alésage 2, la rondelle 10 doit être disposée de manière que son ouverture 18 soit placée au-dessus de l'autre alésage 2 pour permettre la déformation élastique de ladite rondelle, sous l'effort de pression de ladite vis.

Ainsi, la rondelle ouverte 10 du dispositif anti-recul 5 permet de retenir deux vis de fixation 3, du fait de sa position axiale par rapport aux alésages 2.

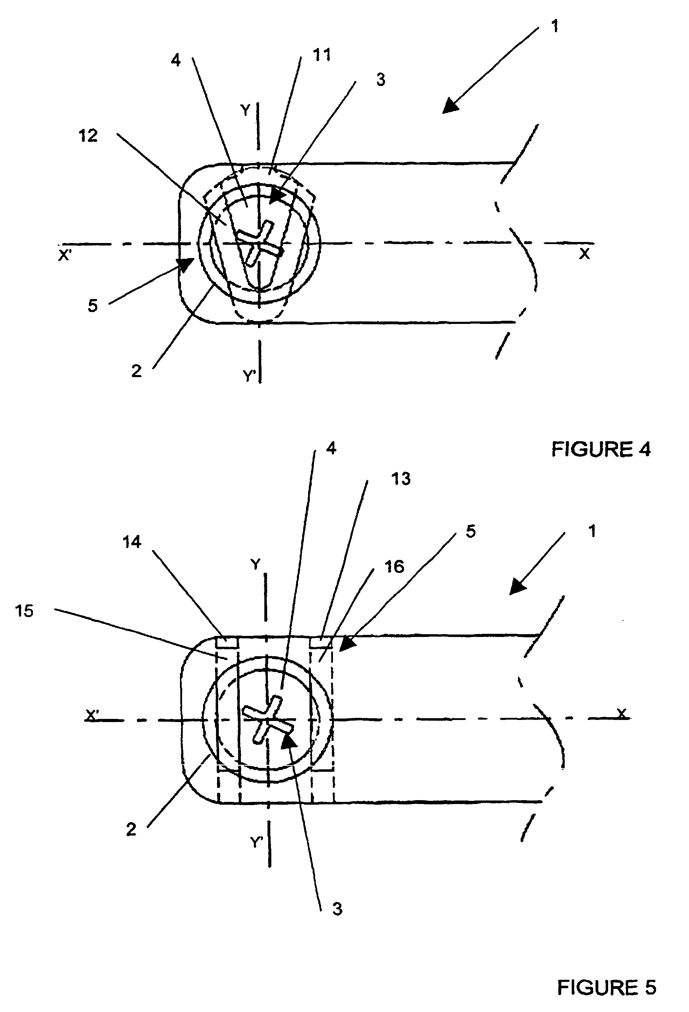

On a montré en figure 4 une seconde variante du dispositif anti-recul 5 suivant la présente invention.

Le dispositif anti-recul 5 comprend un logement qui est constitué d'un espace 11 dont les bords opposés sont inclinés pour présenter un profil en forme de V disposé dans un plan horizontal qui est perpendiculaire à l'axe longitudinal de l'alésage 2 de l'implant orthopédique 1.

L'espace 11 est disposé de manière à être centré autour du point d'intersection des axes principaux XX' et YY' de l'alésage 2.

Le dispositif anti-recul 5 comprend à l'intérieur du logement 11 un moyen de retenue qui est constitué d'une tige 12 en forme de V susceptible de se déformer étastiquement en flexion sous un effort de pression.

La tige 12 est disposée à l'intérieur de l'espace 11 de manière que, de part et d'autre du centre d'insertion des axes XX' et YY', l'alésage 2 soit traversé par les branches inclinées de ladite tige.

Lors de l'introduction de la vis de fixation 3 à l'intérieur de l'alésage 2, et de son ancrage dans l'os suivant une direction angulaire déterminée, l'effort de pression soumis à la tige 12 par la tête de serrage 4 déforme cette dernière, et plus particulièrement chacune de ses branches inclinées à l'intérieur de l'espace 11 pour laisser le passage à ladite vis.

On note, lorsque la vis 3 est complètement introduite dans l'alésage 2 ou ancrée dans l'os du patient, que la tige 12, du fait de son élasticité et du retrait de l'effort de pression, reprend sa position d'origine, non déformée, au-dessus de la tête de serrage 4 pour empêcher ladite vis de se déplacer par rapport à l'implant 1 suivant une direction sensiblement parallèle à celle de l'axe longitudinal de l'alésage 2.

On a montré en figure 5 une troisième variante du dispositif anti-recul 5 suivant la présente invention.

Le dispositif anti-recul 5 comprend dans un même plan horizontal un logement qui est constitué de deux trous parallèles 13 et 14 débouchant à l'intérieur de l'alésage 2 de l'implant orthopédique 1.

Les trous 13, 14 sont prévus pour traverser l'alésage 2 de chaque côté du centre d'insertion des axes XX' et YY', et suivant une direction parallèle à celle de l'axe YY'.

Le dispositif anti-recul 5 comprend à l'intérieur de chaque trou 13, 14 un moyen de retenue qui est constitué d'une tige 15, 16 susceptible de se déformer élastiquement en flexion sous un effort de pression.

Les tiges 15, 16 sont disposées respectivement à l'intérieur des trous 13, 14 de manière à traverser l'alésage 2 de l'implant 1.

Les tiges 15, 16, et les trous 13, 14 du dispositif anti-recul 5 sont disposés dans un même plan horizontal qui est perpendiculaire à l'axe longitudinal de l'alésage 2.

Les tiges 15, 16 traversent l'alésage 2 de l'implant 1 suivant une direction qui est éloignée du centre d'insertion des axes XX' et YY', mais parallèle à l'axe YY' et contenue dans l'espace délimité par la surface circulaire dudit alésage.

En outre, les tiges 15, 16 traversent l'alésage 2 à l'intérieur de sa surface circulaire, mais en bordure de cette dernière.

Lors de l'introduction de la vis de fixation 3 à l'intérieur de l'alésage 2, et de son ancrage dans l'os suivant une direction angulaire déterminée, l'effort de pression soumis aux tiges 15, 16 par la tête de serrage 4 déforme ces dernières en flexion, entraînant un faible déplacement desdites tiges à l'intérieur des trous 13, 14 pour laisser le passage à ladite vis.

On note, lorsque la vis 3 est complètement introduite dans l'alésage 2 ou ancrée dans l'os du patient, que les tiges 15, 16, du fait de leur élasticité et du retrait de l'effort de pression, reprennent leur position d'origine, non déformée, au-dessus de la tête de serrage 4 pour empêcher ladite vis de se déplacer par rapport à l'implant 1 suivant une direction sensiblement parallèle à celle de l'axe longitudinal de l'alésage 2.

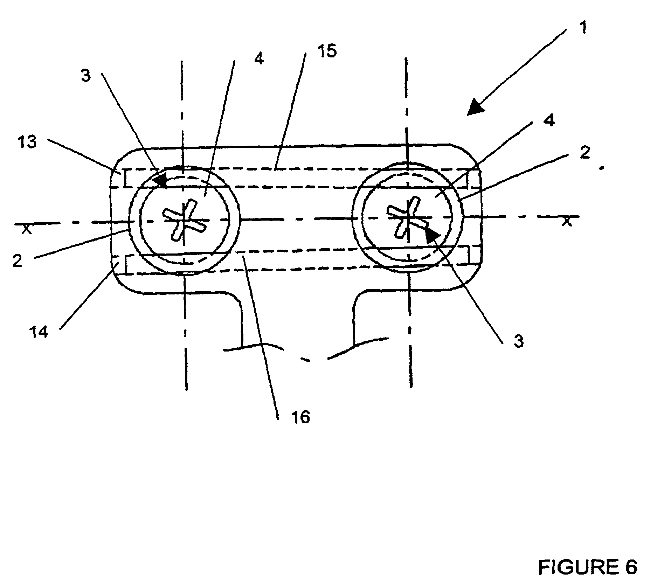

On a montré en figure 6 une quatrième variante du dispositif anti-recul 5 suivant la présente invention qui consiste principalement à disposer les trous 13, 14 et les tiges 15, 16 représentés en figure 5 dans une direction qui est parallèle à l'axe XX' de l'alésage 2.

En outre les mêmes tiges 15, 16 peuvent être utilisées pour plusieurs alésages 2 afin de retenir différentes vis de fixation 3 (figure 6).

Il doit d'ailleurs être entendu que la description qui précède n'a été donnée qu'à titre d'exemple et qu'elle ne limite nullement le domaine de l'invention dont on ne sortirait pas en remplaçant les détails d'exécution décrits par tout autre équivalent

## Revendications

1. Implant orthopédique comprenant au moins un alésage (2) destiné à recevoir une vis de fixation (3) solidaire d'une tête (4) et un dispositif anti-recul (5), **caractérisé en ce que** le dispositif anti-recul (5) comprend :
• au moins un logement (6, 7 ; 17 ; 11 ; 13, 14) qui est décalé axialement par rapport aux axes principaux XX' et YY' de l'alésage (2),
• et au moins un moyen de retenue (8 ; 10 ; 12 ; 15, 16) qui coopère avec le logement correspondant afin que ledit moyen de retenue traverse partiellement l'alésage (2) correspondant recevant la vis de fixation (3) pour pouvoir se déformer élastiquement sous un effort de pression afin de permettre le passage et la mise en place de la vis (3) dans son alésage (2), tandis que le moyen de retenue revient dans une position d'origine, non déformée, lors du retrait de l'effort de pression, pour se positionner au-dessus de la tête (4) de la vis (3) afin d'empêcher cette dernière de se déplacer par rapport à l'implant (1) suivant une direction parallèle à l'axe longitudinal de l'alésage (2).

2. Implant orthopédique suivant la revendication 1, **caractérisé en ce que** le dispositif anti-recul (5) comprend, dans un même plan horizontal, un logement (6) de faible diamètre comportant un espace de débattement (7) débouchant à l'intérieur de l'alésage (2) et un moyen de retenue qui est constitué d'une tige (8) dont une partie est fixée dans le logement (6) de manière à ce que l'autre partie traverse partiellement l'alésage (2) et vienne déboucher à l'intérieur de l'espace de débattement (7) pour se déformer élastiquement en flexion sous un effort de pression.

3. Implant orthopédique suivant la revendication 2, **caractérisé en ce que la** tige (8) traverse l'alésage (2) de l'implant (1) suivant une direction qui est éloignée du point d'intersection des axes XX' et YY'.

4. Implant orthopédique suivant la revendication 2, **caractérisé en ce que** la tige (8), le trou (6) et l'espace (7) sont disposés dans un même plan horizontal qui est perpendiculaire à l'axe longitudinal de l'alésage (2).

5. Implant orthopédique suivant la revendication 1, **caractérisé en ce qu**'il comprend, dans un même plan horizontal, un logement qui est formé d'une rainure circulaire (17) décalée axialement par rapport aux axes principaux XX' et YY' de l'alésage (2) de manière à ce que le moyen de retenue constitué d'une rondelle ouverte (10) traverse partiellement l'alésage (2).

6. Implant orthopédique suivant la revendication 1, **caractérisé en ce que** la position du logement circulaire (17) est prévue pour déboucher dans au moins deux alésages (2) afin de permettre au moyen de retenue constitué d'une rondelle ouverte (10), de traverser partiellement les deux alésages (2).

7. Implant orthopédique suivant la revendication 6, **caractérisé en ce que** la rondelle ouverte (10) est disposée dans le logement (17) de manière que son ouverture (18) soit placée au-dessus de l'un des alésages (2) pour pouvoir se déformer élastiquement.

8. Implant orthopédique suivant la revendication 1, **caractérisé en ce qu**'il comprend, dans un même plan horizontal, un logement qui est formé d'un espace (11) dont les bords opposés sont inclinés pour présenter un profil en forme de V et un moyen de retenue qui est constitué d'une tige (12) de même profil introduite dans l'espace (11) en forme de V pour se déformer élastiquement en flexion sous un effort de pression.

9. Implant orthopédique suivant la revendication 8, **caractérisé en ce que** la tige (12) est disposée à l'intérieur de l'espace (11) de manière que, de part et d'autre du point d'insertion des axes XX' et YY', l'alésage (2) soit traversé partiellement par les branches inclinées de ladite tige.

10. Implant orthopédique suivant la revendication 1, **caractérisé en ce qu**'il comprend, dans un même plan horizontal, un logement qui est formé de deux trous parallèles (13, 14) débouchant à l'intérieur de l'alésage (2) de l'implant (1) et un moyen de retenue qui est constitué de deux tiges (15, 16) coopérant respectivement avec les trous (13, 14) pour se déformer élastiquement en flexion sous un effort de pression.

11. Implant orthopédique suivant la revendication 10, **caractérisé en ce que** les trous (13, 14) sont prévus pour traverser l'alésage (2) de chaque côté du point d'intersection des axes principaux XX' et YY' et suivant une direction parallèle à celle de l'axe YY'.

12. Implant orthopédique suivant la revendication 10, **caractérisé en ce que** les trous (13, 14) sont prévus pour traverser l'alésage (2) de chaque côté du point d'intersection des axes principaux XX' et YY' et suivant une direction parallèle à celle de l'axe XX'.

## Patentansprüche

1. Orthopädisches Implantat, das mindestens eine Bohrung (2), die dazu bestimmt ist, eine fest mit einem Kopf (4) verbundene Befestigungsschraube (3) aufzunehmen, und eine rückzugsverhindernde Vorrichtung (5) aufweist, **dadurch gekennzeichnet, dass** die rückzugsverhindernde Vorrichtung (5) aufweist:
- mindestens einen Sitz (6, 7; 17; 11; 13, 14), der axial bezüglich der Hauptachsen XX' und YY' der Bohrung (2) versetzt ist,
- und mindestens ein Rückhaltemittel (8; 10; 12; 15, 16), das mit dem entsprechenden Sitz zusammenwirkt, damit das Rückhaltemittel teilweise die entsprechende die Befestigungsschraube (3) aufnehmende Bohrung (2) überquert, um sich elastisch unter einer Druckkraft verformen zu können, damit der Durchlass und das Einsetzen der Schraube (3) in ihre Bohrung (2) ermöglicht werden, während das Rückhaltemittel bei Nachlassen der Druckkraft in eine nicht verformte Ursprungsstellung zurückkommt, um sich oberhalb des Kopfes (4) der Schraube (3) zu positionieren, damit letztere daran gehindert wird, sich bezüglich des Implantats (1) in einer Richtung parallel zur Längsachse der Bohrung (2) zu verschieben.

2. Orthopädisches Implantat nach Anspruch 1, **dadurch gekennzeichnet, dass** die rückzugsverhindernde Vorrichtung (5) in der gleichen waagrechten Ebene einen Sitz (6) mit geringem Durchmesser, der einen Ausfederungsraum (7) besitzt, der innerhalb der Bohrung (2) mündet, und ein Rückhaltemittel aufweist, das aus einer Stange (8) besteht, von der ein Teil am Sitz (6) so befestigt ist, dass der andere Teil die Bohrung (2) teilweise überquert und innerhalb des Ausfederungsraums (7) mündet, um sich unter der Wirkung einer Druckkraft elastisch zu verformen.

3. Orthopädisches Implantat nach Anspruch 2, **dadurch gekennzeichnet, dass** die Stange (8) die Bohrung (2) des Implantats (1) in einer Richtung überquert, die vom Schnittpunkt der Achsen XX' und YY' entfernt ist.

4. Orthopädisches Implantat nach Anspruch 2, **dadurch gekennzeichnet, dass** die Stange (8), das Loch (6) und der Raum (7) sich in der gleichen waagrechten Ebene befinden, die senkrecht zur Längsachse der Bohrung (2) liegt.

5. Orthopädisches Implantat nach Anspruch 1, **dadurch gekennzeichnet, dass** es in der gleichen waagrechten Ebene einen Sitz aufweist, der von einer kreisförmigen Nut (17) gebildet wird, die bezüglich der Hauptachsen XX' und YY' der Bohrung (2) axial so versetzt ist, dass das aus einer offenen Scheibe (10) bestehende Rückhaltemittel teilweise die Bohrung (2) durchquert.

6. Orthopädisches Implantat nach Anspruch 1, **dadurch gekennzeichnet, dass** die Stellung des kreisförmigen Sitzes (17) so vorgesehen ist, dass er in mindestens zwei Bohrungen (2) mündet, um es dem aus einer offenen Scheibe (10) bestehenden Rückhaltemittel zu erlauben, die beiden Bohrungen (2) teilweise zu überqueren.

7. Orthopädisches Implantat nach Anspruch 6, **dadurch gekennzeichnet, dass** die offene Scheibe (10) im Sitz (17) so angeordnet ist, dass ihre Öffnung (18) sich oberhalb einer der Bohrungen (2) befindet, um sich elastisch verformen zu können.

8. Orthopädisches Implantat nach Anspruch 1, **dadurch gekennzeichnet, dass** es in der gleichen waagrechten Ebene einen Sitz, der von einem Raum (11) gebildet wird, dessen gegenüberliegende Ränder geneigt sind, um ein V-förmiges Profil zu bilden, und ein Rückhaltemittel aufweist, das aus einer Stange (12) mit gleichem Profil besteht, die in den V-förmigen Raum (11) eingeführt ist, um sich unter einer Druckkraft elastisch zu verbiegen.

9. Orthopädisches Implantat nach Anspruch 8, **dadurch gekennzeichnet, dass** die Stange (12) im Inneren des Raums (11) derart angeordnet ist, dass die Bohrung (2) zu beiden Seiten des Schnittpunkts der Achsen XX' und YY' teilweise von den geneigten Schenkeln der Stange überquert wird.

10. Orthopädisches Implantat nach Anspruch 1, **dadurch gekennzeichnet, dass** es in der gleichem waagrechten Ebene einen Sitz, der von zwei parallelen Löchern (13, 14) gebildet wird, die im Inneren der Bohrung (2) des Implantats (1) münden, und ein Rückhaltemittel aufweist, das von zwei Stangen (15, 16) gebildet wird, die je mit den Löchern (13, 14) zusammenwirken, um sich unter einer Druckkraft elastisch zu verbiegen.

11. Orthopädisches Implantat nach Anspruch 10, **dadurch gekennzeichnet, dass** die Löcher (13, 14) vorgesehen sind, um die Bohrung (2) auf jeder Seite des Schnittpunkts der Hauptachsen XX' und YY' und in einer Richtung parallel zu derjenigen der Achse YY' zu überqueren.

12. Orthopädisches Implantat nach Anspruch 10, **dadurch gekennzeichnet, dass** die Löcher (13, 14) vorgesehen sind, um die Bohrung (2) auf jeder Seite des Schnittpunkts der Hauptachsen XX' und YY' und in einer Richtung parallel zu derjenigen der Achse XX' zu überqueren.

## Claims

1. Orthopaedic implant comprising at least one bore (2) intended to receive a fixation screw (3) integral with a head (4) and an anti-slip device (5), **characterized in that** the anti-slip device (5) comprises:
• at least one seat (6, 7; 17; 11; 13, 14) which is offset axially relative to the main axes XX' and YY' of the bore (2),
• and at least one retaining means (8; 10; 12; 15, 16) which cooperates with the corresponding seat so that said retaining means passes partially through the corresponding bore (2) receiving the fixation screw (3) in order to be able to deform elastically under a pressure stress in order to allow the screw (3) to be passed into and installed in its bore (2), while the retaining means returns to an original, non-deformed, position upon removal of the pressure stress, so as to be located above the head (4) of the screw (3) in order to prevent the latter from being displaced relative to the implant (1) in a direction parallel to the longitudinal axis of the bore (2).

2. Orthopaedic implant according to Claim 1, **characterized in that** the anti-slip device (5) comprises, in the same horizontal plane, a seat (6) of small diameter comprising a clearance space (7) opening inside the bore (2) and a retaining means which is formed by a rod (8), one part of which is fixed in the seat (6) in such a way that the other part passes partially through the bore (2) and opens out inside the clearance space (7) in order to deform elastically in flexion under a pressure stress.

3. Orthopaedic implant according to Claim 2, **characterized in** the rod (8) passes through the bore (2) of the implant (1) in a direction which is remote from the point of intersection of the axes XX' and YY'.

4. Orthopaedic implant according to Claim 2, **characterized in that** the rod (8), the hole (6) and the space (7) are arranged in the same horizontal plane which is perpendicular to the longitudinal axis of the bore (2).

5. Orthopaedic implant according to Claim 1, **characterized in that** it comprises, in the same horizontal plane, a seat which is formed by a circular groove (17) offset axially relative to the main axes XX' and YY' of the bore (2) in such a way that the retaining means formed by an open washer (10) passes partially through the bore (2).

6. Orthopaedic implant according to Claim 1, **characterized in that** the position of the circular seat (17) is intended to open into at least two bores (2) in order to allow the retaining means formed by an open washer (10) to pass partially through the two bores (2).

7. Orthopaedic implant according to Claim 6, **characterized in that** the open washer (10) is arranged in the seat (17) in such a way that its opening (18) is placed above one of the bores (2) so as to be able to deform elastically.

8. Orthopaedic implant according to Claim 1, **characterized in that** it comprises, in the same horizontal plane, a seat which is formed by a space (11) whose opposite edges are inclined in order to present a V-shaped profile and a retaining means which is formed by a rod (12) of the same profile introduced into the V-shaped space (11) in order to deform elastically in flexion under a pressure stress.

9. Orthopaedic implant according to Claim 8, **characterized in that** the rod (12) is arranged inside the V-shaped space (11) so that, on both sides of the point of intersection of the axes XX' and YY', the bore (2) is traversed at least partially by the inclined branches of said rod.

10. Orthopaedic implant according to Claim 1, **characterized in that** it comprises, in the same horizontal plane, a seat which is formed by two parallel holes (13, 14) opening inside the bore (2) of the implant (1) and a retaining means which is formed by two rods (15, 16) cooperating respectively with the holes (13, 14) in order to deform elastically in flexion under a pressure stress.

11. Orthopaedic implant according to Claim 10, **characterized in that** the holes (13, 14) are provided to pass through the bore (2), each side of the point of intersection of the main axes XX' and YY' and in a direction parallel to that of the axis YY'.

12. Orthopaedic implant according to Claim 10, **characterized in that** the holes (13, 14) are provided to pass through the bore (2), each side of the point of intersection of the main axes XX' and YY' and in a direction parallel to that of the axis XX'.
